Europäisches Patentamt

European Patent Office

Office européen des brevets

(11) Publication number: **0 253 927**
**A1**

# (12) EUROPEAN PATENT APPLICATION

(21) Application number: **86303715.6**

(22) Date of filing: **15.05.86**

(51) Int. Cl.⁴: **G01N 33/497 ,**
**//C12Q1/58,G01N33/62,A61K49-**
**/00**

The title of the invention has been amended (Guidelines for Examination in the EPO, A-III, 7.3).

(30) Priority: **21.04.86 US 854361**

(43) Date of publication of application:
**27.01.88 Bulletin 88/04**

(84) Designated Contracting States:
**CH DE FR GB IT LI NL SE**

(71) Applicant: **Baylor College of Medicine**
**Texas Medical Center 1200 Moursund**
**Avenue**
**Houston Texas 77030(US)**

(72) Inventor: **Klein, Peter D.**
**2710 Glenhaven Boulevard**
**Houston Texas 77025(US)**
Inventor: **Graham, David Y.**
**4051 Mishire**
**Houston Texas 77025(US)**

(74) Representative: **Wilkinson, Stephen John et al**
**c/o Stevens, Hewlett & Perkins 5 Quality**
**Court Chancery Lane**
**London WC2A 1HZ(GB)**

(54) A breath test for measuring urease activity in the stomach using carbon isotope urea.

(57) A procedure for a non-invasive breath test for measuring urease activity in the stomach and areas proximate thereto, the test including feeding isotopical carbon-labeled urea to a test subject, collecting a breath sample or samples from the test subject, analyzing the sample for the presence of the labeling isotope in exhaled carbon dioxide and also, in one embodiment, measuring the amount of labeling isotope present and determining the amount of the isotope which originated within the labeled urea.

EP 0 253 927 A1

# A PROCEDURE FOR A NON-INVASIVE BREATH TEST FOR MEASURING UREASE ACTIVITY IN THE STOMACH AND AREAS PROXIMATE THERETO USING CARBON ISOTOPE-LABELED UREA AND MATERIAL USEFUL IN THE TEST

## BACKGROUND OF THE INVENTION

### Field of the Invention

This invention relates to a non-invasive breath test measuring urease activity in the stomach and proximal intestine and for detecting the presence of microorganisms that produce urease such as pyloric campylobacter; particularly to such a test which utilizes urea labeled with a carbon isotope as a probe substrate; and to materials useful in performing such a test.

### Description of the Prior Art

Many efforts have been made to understand and treat the common afflictions of dyspepsia and gastritis. Advances in medical knowledge have resulted in a number of schemes to classify dyspepsia; currently patients are separated into those with dyspepsia secondary to peptic ulcer and those with non-ulcer dyspepsia.

It is known that campylobacter-like bacteria, (generally termed "CLO" or "pyloric campylobacter") are present in the stomach of most patients with gastric or duodenal ulcers, in those with non-ulcer dyspepsia, and in those with gastritis. The relationship of these bacteria to the pathogenesis of the ulcer disease, gastritis or the symptoms associated with ulcer or non-ulcer dyspepsia is still unclear. However, there is circumstantial evidence which suggests that CLO may play a role in human disease. For example, CLO organisms are killed by bismuth compounds and it is known that a bismuth preparation is effective both in healing peptic ulcer and in the prevention of ulcer relapse. More importantly, after therapy with bismuth is stopped, the natural history of the ulcer appears changed, i.e., the relapse rate is less than if the patient had received another form of therapy. This suggests that there has been a fundamental change of conditions in the stomach.

The relationship of CLO to gastritis has been demonstrated by an investigator who has reported the results of his deliberate ingestion of an suspension of CLO organisms. The investigator developed a symptomatic gastritis and was able to recover CLO organisms from his own gastric biopsy.

In the past, it has been very difficult to establish that CLO organisms are present in the stomach. CLO organisms attach themselves to the gastric mucosa and are also found within the mucosa. This localization means that a gastric biopsy must be carried out so that the presence of CLO can be confirmed by special staining or culture of the biopsy specimen incubating it in a 2% urea broth for 24 hours and measuring the color change from brown to pink (McNulty, C.A.M. and Wise, R. Rapid diagnosis of campylobacter-associated gastritis Lancet i: 1443-1444, 1985). Measures to quantify infestation are difficult to derive from such qualitative tests. There is great need for a simple, rapid and non-invasive procedure for determining the presence and quantity of bacterial urease present in the stomach and upper small intestine as a means identifying the presence of CLO organisms in the stomach.

## SUMMARY OF THE INVENTION

The present invention is directed to a non-invasive breath test using carbon-isotopically-labeled urea to identify the presence of, and localize to the stomach and proximal intestine, microorganisms that produce urease. Urease splits urea into carbon dioxide and ammonia. Urea labeled with a non-radioactive isotope of carbon such as $^{13}C$ or a radioactive isotope such as $^{11}C$ or $^{14}C$ is employed. It is preferred that the interaction of the substrate with the urease-producing microorganisms be prolonged. This is accomplished by the use of a nutrient-dense meal that delays gastric emptying and the transit of the substrate through the small intestine. The test procedure also employs a means of detecting hydrolysis of the substrate by the increase in the isotopic carbon content of respiratory carbon dioxide. The prompt appearance of labeled carbon dioxide in breath confirms the site of urease activity is in the stomach and/or areas proximate thereto such as the proximal small intestine. In individuals with pyloric campylobacter infestation, urea-derived carbon dioxide appears in breath within 20 minutes and accumulates at a constant rate for more than 100 minutes while normal test subjects show no release of labeled carbon dioxide. Certain calculations can be performed that permit quantification of the degree of bacterial infestation in addition to qualitative establishment of its presence.

Since the test is non-invasive, and since the preferred substrate label is the stable, non-radioactive isotope $^{13}C$, this test may be used without hazard in:

(1) infants, children, pregnant or lactating women;

(2) repeated observations in the same individual; and

(3) surveys of populations who may be at risk, but which show no symptoms warranting the invasive use of gastric biopsy.

It is, therefore, an object of this invention to provide a new and useful test for demonstrating the presence of bacterial urease in the stomach and upper small intestine. Another object is the provision of such a test for the assessment of the presence of pyloric campylobacter.

Another object of the present invention is the provision of such a test which is non-invasive, such as a breath test.

Yet another object of the present invention is the provision of such a test which uses ingested urea which reacts with bacterial urease to form inter alia, carbon dioxide which is exhaled and can be collected and measured.

Still another object of the present invention is the provision of such a test in which a pharmaceutically-acceptable nutrient-dense meal is ingested by the test subject to prevent gastric emptying so that the interaction of the urease-organism with the urea is prolonged.

A particular object of the present invention is the provision of the pharmaceutically-acceptable nutrient-dense meal.

Another particular object is the provision of such a meal containing the urea substrate.

An additional object of the present invention is the provision of such a test in which the urea is labeled with an excess of a carbon isotope.

A particular object of the present invention is the provision of such a test in which the carbon isotope is a non-radioactive carbon isotope.

Another particular object of the present invention is the provision of such a test in which a radioactive carbon isotope is used.

Another object of the present invention is the provision of a breath test which provides quantitative information of the degree of bacterial infestation.

Other and further objects, features, and advantages will be clear to one of skill in this art (who has the benefit of this invention's teachings) from the following description of presently preferred embodiments of the invention, given for the purpose of disclosure. When taken in conjunction with the accompanying figures.

DESCRIPTION OF THE DRAWINGS

TABLE I lists the values for the cumulative percent dose of $^{13}C$ urea recovered as $^{13}CO_2$ in 21 studies according to the present invention as a function of time after administration together with results of gastric biopsies stained, examined and cultured for the presence of campylobacter pyloridis.

FIG. 1A is a graph showing the $^{13}C$ isotopic abundance of breath (measured relative to an established reference standard) from a normal subject for 30 minutes before and 180 minutes after ingestion of $^{13}C$-labeled urea (5 mg/kg body weight) in a test according to the present invention.

FIG. 1B is the same information as that of FIG. 1 recalculated to show excess (greater than normal) concentration of $^{13}C$ in units of milli atom percent excess (mAPE).

FIG. 2 is a graph with data derived from a test according to the present invention showing the excess $^{13}C$ concentration in respiratory $CO_2$ following ingestion of 5 mg/kg body weight of $^{13}C$ labeled urea by a subject infested with pyloric campylobacter.

FIGS. 3A and 3B show the cumulative percent dose recovered as $^{13}CO_2$ as a function of time after substrate ingestion according to the present invention for normal and infected subjects.

FIG. 4 shows the association of positive and negative biopsy results with the magnitude of the cumulative percent dose recovered within 120 minutes of substrate administration according to the test of the present invention.

FIG 5 shows the results of testing, according to the present invention, each of 16 individuals twice at intervals of 7-15 days with the repeated observations of each individual shown as connected with horizontal lines.

FIGS. 6A and 6B show the consequence (in a normal individual) of omitting (FIG. 6A) and including (FIG. 6B) a nutrient-dense meal in the performance of the test according to the present invention.

FIG. 7 shows the effect of bismuth therapy on the cumulative percent dose recovered from an individual with proven pyloric campylobacter infestation.

DESCRIPTION OF PREFERRED EMBODIMENTS

The test according to the present invention was administered to 20 subjects after the subjects fasted overnight. One or more baseline samples of the subjects' respiratory $CO_2$ were collected by exhalation through a personal mouthpiece into plastic bags made of a $CO_2$-impervious film. The baseline samples record the starting or natural level of carbon isotopes in breath. Each subject received and consumed a 150 gram portion of a commercial nutrient-dense meal containing 7 grams of protein, 32 grams of carbohydrate and 9.5 grams of fat with a total of 240 calories. Each subject then drank 100 milliliters of water containing a dose of 5 mg/kg weight of $^{13}C$ urea (99% $^{13}C$, MS-2931, MSD Isotopes, Merck Chemical Division, 4545 Oleatha Avenue, St. Louis, MO 63116). Although in this particular test the nutrient-dense meal contained 240 calories, and although a meal with as little as about 75 calories can be used, according to the present invention it is preferred that the meal have at least about 100 calories and that it not contain carbon dioxide. The meal may be in liquid or solid form. For example commercially available SUSTACAL (Trademark) nutrient may be used or a 20% liquid glucose solution. The urea substrate may be combined with the meal for administration as a single material. The preferred dose of urea is 5 mg/kg subject weight, but urea amounts ranging between about 2.5 to about 10 milligrams per kilogram of subject weight are acceptable.

Breath samples were collected from the subjects at 10 minute intervals for 180 minutes. Significant diagnostic information is available within about 2 hours, but high levels of urease and therefore high levels of infestation are apparent in about 30 minutues. $^{13}C$ abundance in respiratory $CO_2$ was measured by automated gas isotope ratio mass spectrometry (Schoeller, D.A. and Klein, P.D. A microprocessor controlled mass spectrometer for the fully automated purification and isotopic analysis of breah $CO_2$. Biomed. Mass Spectrom. 6:350-355, 1979) or by infrared heterodyne ratiometry (Irving, C.S., Klein, P.D., Navratil, P.R. and Boutton, T.W. Measurement of $^{13}CO_2/^{12}CO_2$ abundances by nondispersive infrared heterodyne ratiometry as an alternative to gas isotope ratio mass spectrometry. Anal. Chem. In press 1986).

Excess $^{13}C$ abundance was calculated by the increase in carbon isotope content of the respiratory carbon dioxide over the baseline values obtained before substrate administration. (Schoeller D.A., Schneider, J.F., Solomons, N.W., Watkins, J.B. and Klein, P.D. Clinical diagnosis with the stable isotope $^{13}C$ in $CO_2$ breath tests: Methodology and fundamental considerations J. Lab. Clin. Med. 90:412-421, 1977). $CO_2$ production rates were taken to be 5 millimoles of $CO_2$ per minute per meter body surface area. Body surface area was calculated according to the formula: $M^2 = $ [Body weight (kg)]$^{0.5378}$ x [height (cm)]$^{0.3964}$ x. 0.024265. Thus for example a subject weight 65.9 kg and having a height of 173 cm had a body surface area of 1.78 cm and a $CO_2$ production rate of 8.71 millimoles per minute. The product of $CO_2$ produc-

tion and $^{13}C$ excess abundance at each time point was converted to percent dose recovered and the values were added in a cumulative manner (Watkins, J.B., Klein, P.D., Schoeller, D.A., Kirschner, B.S., Park, R. and Perman, J.A. Diagnosis and differentiation of fat malabsorption in children using $^{13}C$-labeled lipids: Tricotanoin, triolein and palmitic acid breath tests. Gastroenterology 92 911-917, 1982).

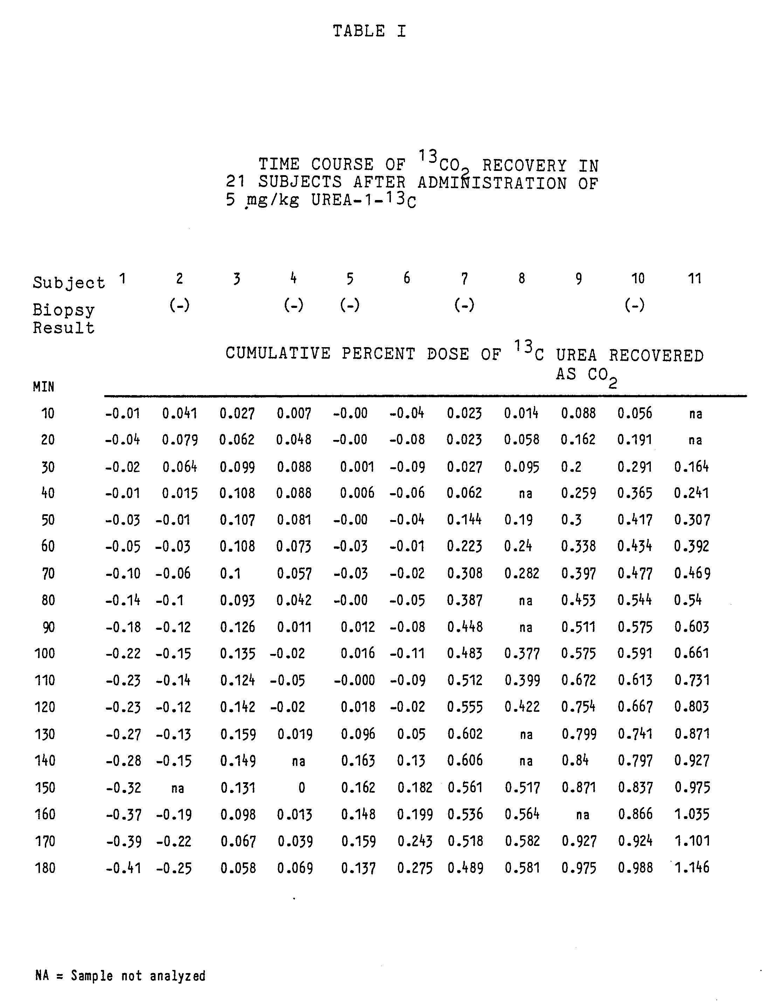

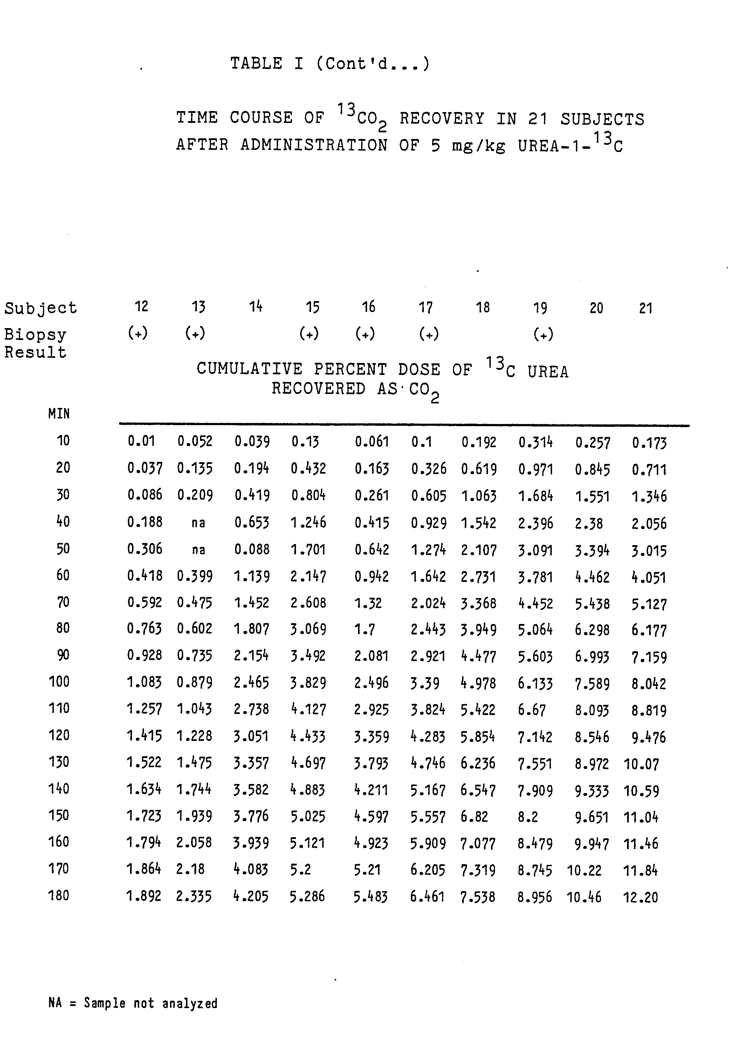

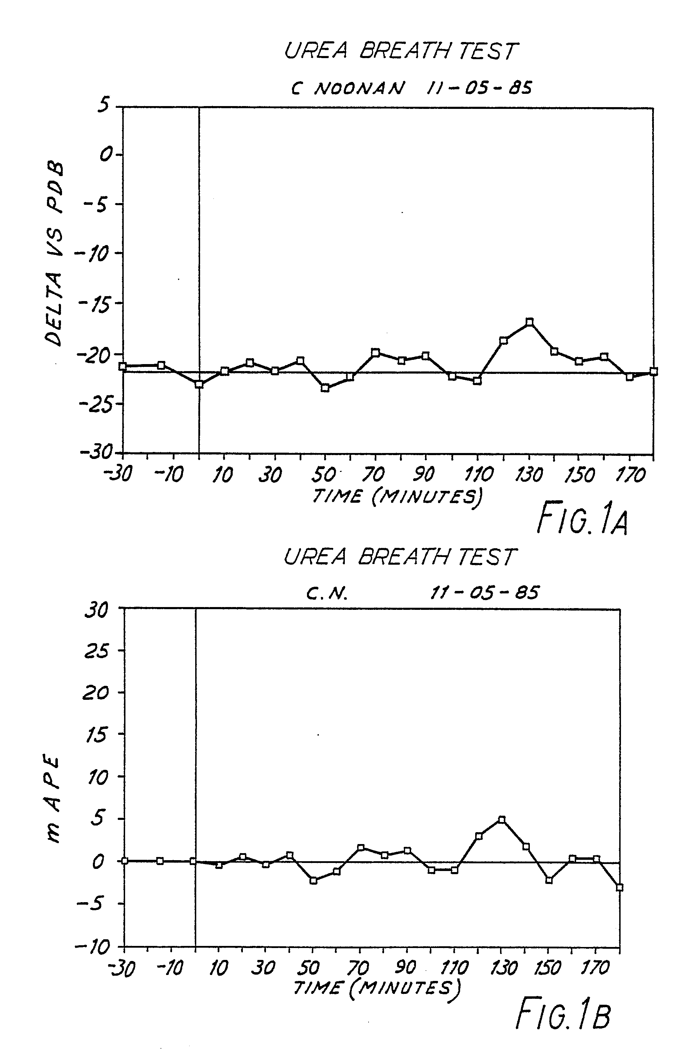

FIG. 1A shows the raw data obtained by gas isotope ratio mass spectrometric analysis of the respiratory carbon dioxide samples. The time in minutes at which the samples are collected is shown on the x axis and the isotopic abundance of $^{13}C$ is shown in the y axis. The units of abundance are in parts per thousand (per mil) difference from the standard reference value PDB. This scale an increase in $^{13}C$ abundance results in a negative number. Because this method of expressing abundance is used chiefly in geochemical studies, the data are transformed in FIG. 1B to yield the excess $^{13}C$ as atom percent excess x $10^{-3}$ or milli atom percent excess. In this notation, the abundance of $^{13}C$ in breath prior to the administration of substrate is selected as the reference point and is assigned the value of zero. In this subject, FIG. 1A, the abundance of $^{13}C$ in respiratory $CO_2$ shows small fluctuations over the first 110 minutes and a significant (greater than 2 mAPE) though small increase thereafter. This normal subject illustrates that when the urea-containing substrate passes down the intestinal tract until the colon is reached, colonic urease-producing organisms may act to produce labeled carbon dioxide, but the transit time required is usually greater than 90-120 minutes. This is indicated by the appearance of labeled $CO_2$ in the time points <u>after</u> 110 minutes. Moreover, the substrate has been diluted and dispersed during its passage down the intestinal tract so that the rate at which labeled $CO_2$ is produced is substantially reduced.

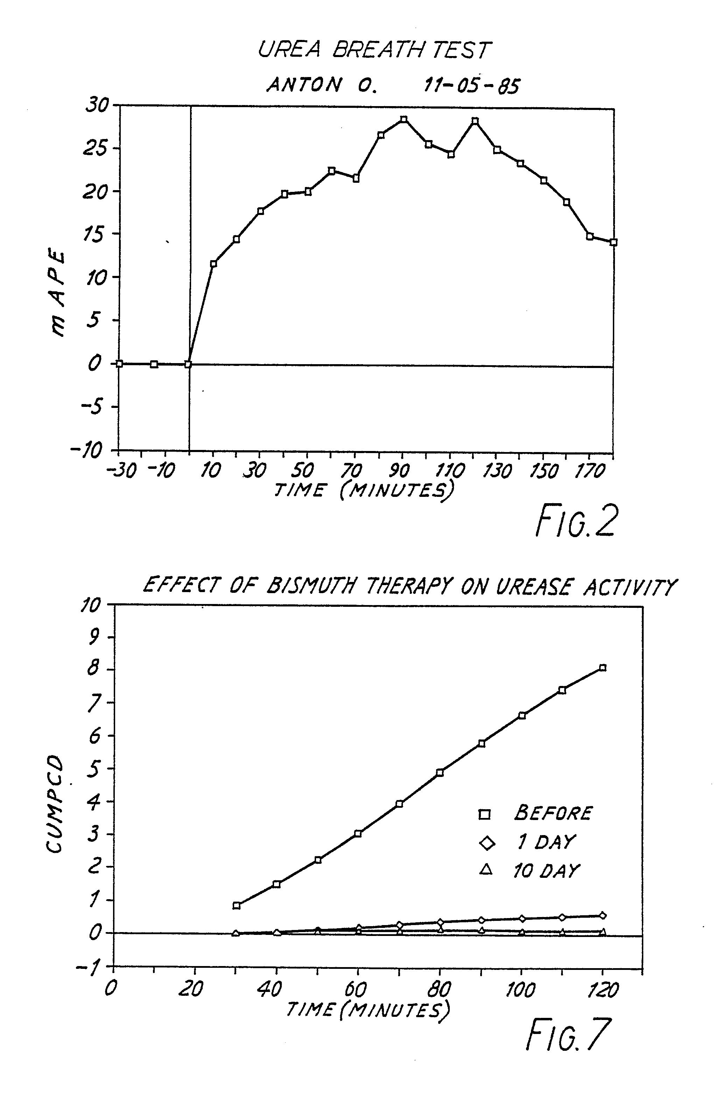

On the other hand, a subject shown by subsequent biopsy and culture to have pyloric campylobacter displays a prompt and extensive excretion of excess $^{13}C$ in his respiratory $CO_2$ as shown in FIG. 2 which illustrates that the urea is acted upon by the urease within a very few minutes after ingestion and must therefore be located in the upper portion of the gastrointestinal tract, i.e., the stomach and proximal small intestine.

FIGS. 1B and 2 demonstrate that the presence of bacterial urease in the stomach and upper small intestine can be demonstrated by external measurments not requiring invasive procedures such as endoscopy and biopsy. In addition to such qualitative information, these data can be further analyzed to provide quantitiative estimations of bacterial infestation.

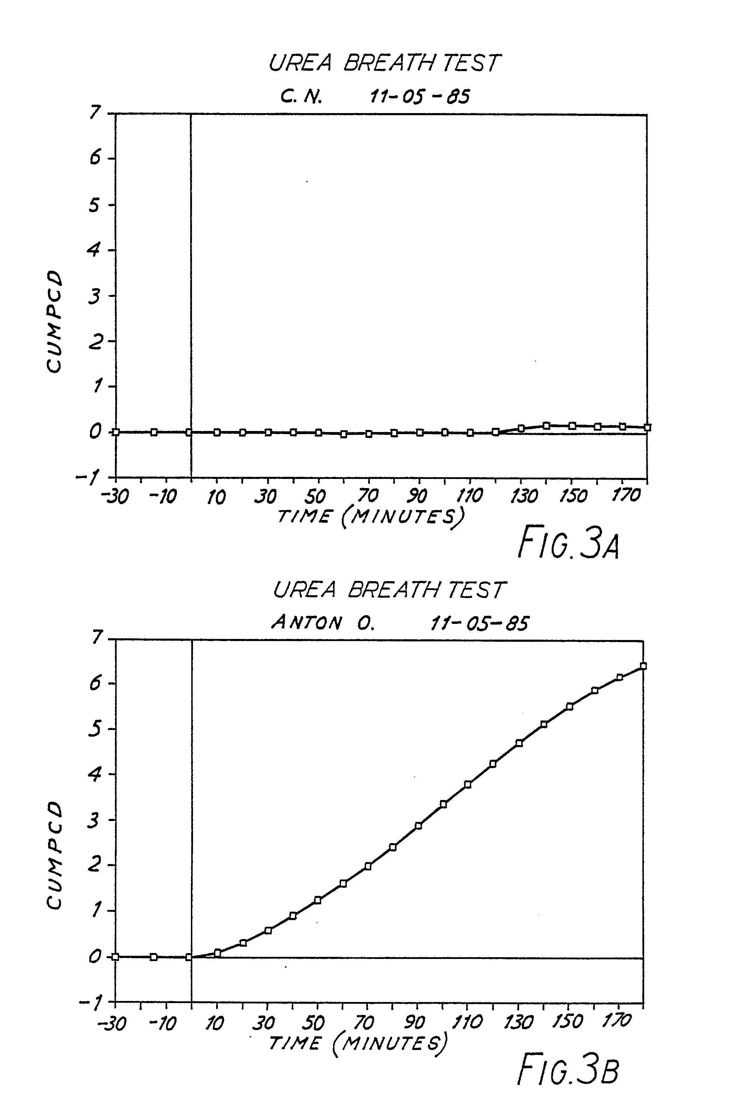

When the cumulative percent dose excreted (corresponding to the total amount of the substrate that has been decomposed by the bacterial urease) is plotted against the time elapsed after substrate administration, the points for the two subjects shown previously in FIGS. 1B and 2 are transformed into those shown in FIGS. 3A and 3B. The specific advantages of this transformation are that: (1) the contribution of individual fluctuations is reduced; and (2) in the presence of bacterial urease activity, the evoluation of $^{13}CO_2$ shows an initial lag (representing absorption and transport of the labeled $CO_2$ to the lungs) followed by a linear rate of production. From this transformation the integrated enzyme activity over a specific period of time (e.g. 120 minutes) can be calculated, or, an expression of enzyme activity in terms of millimoles of urea decomposed per minute can be obtained. Either of these numbers can be used to quantify the level of bacterial infestation, changes in the severity of the disease, and the response to therapeutic measures.

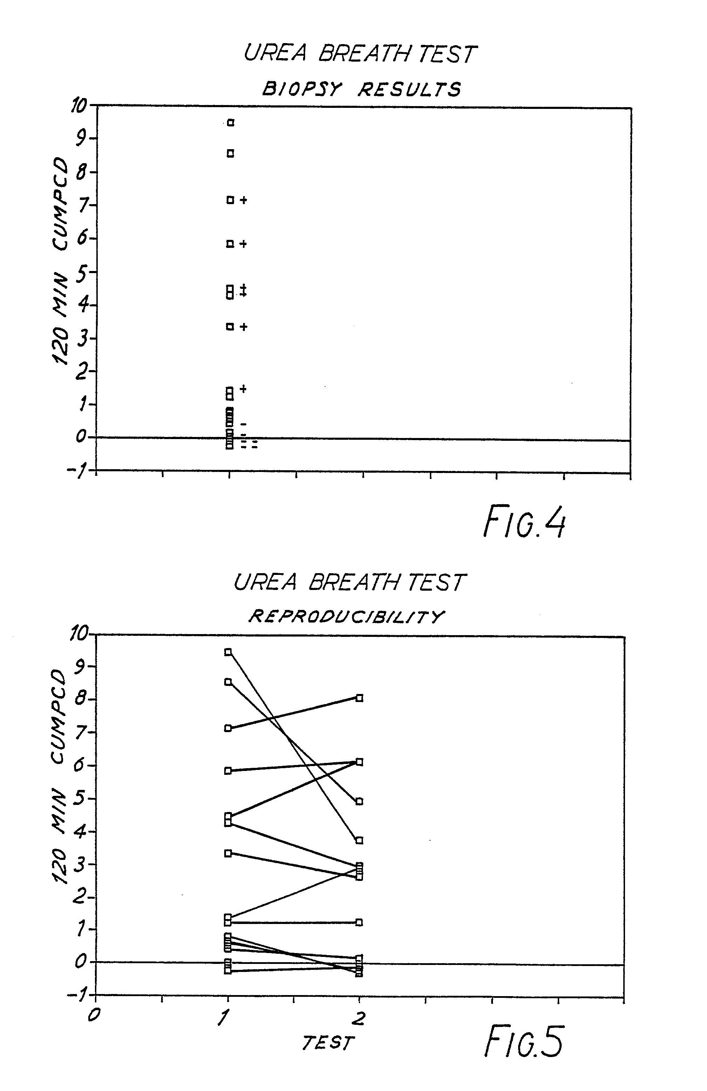

Tabular data for 21 tests is presented in Table I. Under each subject number is listed the result of endoscopic examination, biopsy and culture (if performed) and the cumulative percent dose recovered at 10 minute intervals for 180 minutes. The tests are arranged in order of increasing substrate recovery, not in the order in which the studies were executed. Inspection of the data shows that very little label is recovered in the first 20 minutes and that by 120 minutes the rank order of increasing recovery has been clearly established. Thus the data between the two horizontal lines, constituting half of the total samples analyzed, contain all values needed to characterize the response. The cumulative percent recovery at 120 minutes is shown in rank order in FIG. 4 together with the biopsy results then available. All positive biopsy results were associated with cumulative percent recoveries that were greater than those of all negative biopsy subjects. The highest recover in a negative subject was 0.66% and the lowest positive result was 1.22%. In general, all subjects with positive cultures had cumulative percent recoveries well above 2%.

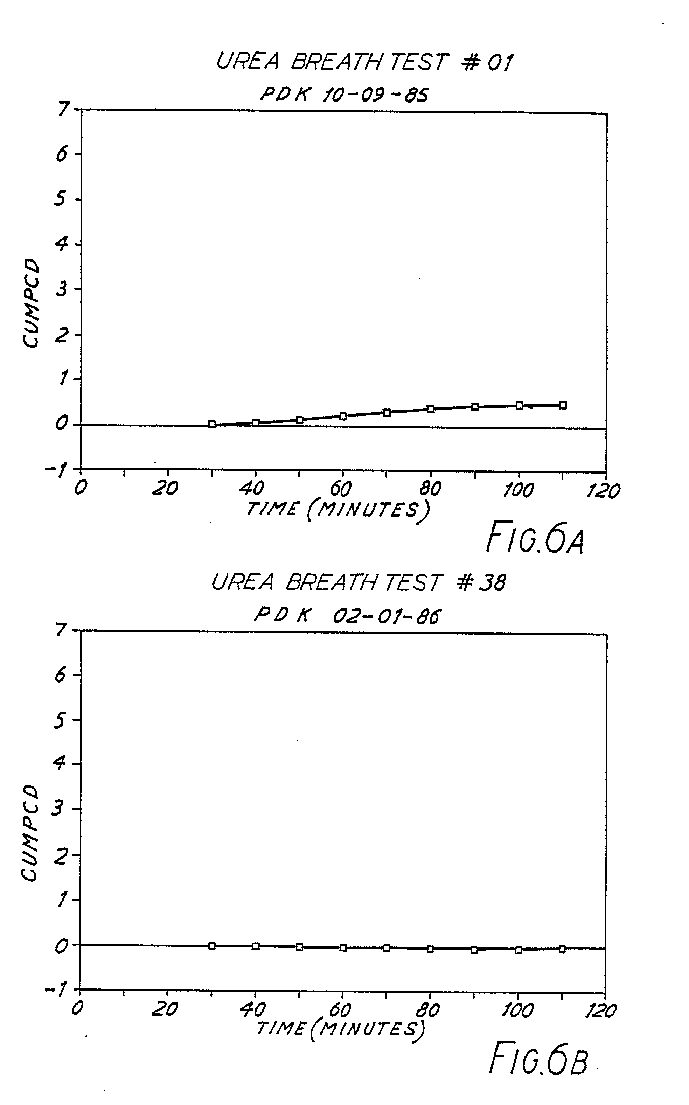

Those individuals who had positive results on their first test continued to show substantial urease activity when retested on a subsequent date, while those who had low levels originally did not change (FIG. 5). This illustrates that the test is repeatable and does not reflect transient appearances of bacterial urease activity.

The role of the nutrient-dense meal in delaying passage of the substrate through the intestinal tract is shown for the recovery of labeled $CO_2$ in a normal subject without (FIG. 6A) and with (FIG. 6B) the test meal. Escape of the substrate into the colon is seen in the upper figure and is absent in the lower figure. In cases of borderline bacterial infestation, resolution of positive and negative subjects is enhanced by the nutrient-dense meal not only because of the longer gastric exposure of the substrate, but also by retardation of its arrival in the colon.

The ability of a test according to the present invention to reflect changes in bacterial infestation is shown by the results in FIG. 7 which record three successive tests in the same individual: before therapy after 1 day and after 10 days of bismuth therapy (commercially available over-the-counter bismuth subnitrate, 2 tablets, 4 time per day). Before therapy pronounced levels of bacterial urease were detectable in the cumulative percent dose recovered over 120 minutes. Within 1 day of therapy, this recovery fell to normal values and remained normal after continuation of the therapy for 10 days. This illustrates the use of this test to monitor patient status over time and in response to therapy or exposure to the causative organism.

In conclusion, therefore, it is seen that the present invention and the embodiments disclosed herein are well adapted to carry out the objectives and obtain the ends set forth. To one of skill in this art who has the benefit of this invention's teachings it will be clear that certain changes can be made in the procedure and apparatus without departing from the spirit of this invention and its scope as defined in the following claims.

## Claims

1. A breath test procedure for detecting urease activity in a test subject's stomach, the procedure comprising
consumption by the subject of urea labeled with a labeling carbon isotope;
collection of one or more test breath samples from the subject after the subject has consumed the urea, and
analysis of the one or more test breath samples for carbon dioxide containing the carbon isotope.

2. The procedure of claim 1 including determining the amount of the carbon isotope in the test breath sample.

3. The procedure of claim 2 including establishing baseline amount of the labeling carbon isotope naturally occurring in the test subject's breath prior to any activity of the labeled urea in the test subject's stomach.

4. The procedure of claim 3 wherein the baseline amount is determined by taking a pre-test sample from the test subject and analyzing it to determine the amount of naturally occurring labeling carbon isotope in the sample.

5. The procedure of claim 3 including determining the amount of labeling carbon isotope in the test breath sample.

6. The procedure of claim 1 in which the more than one test breath samples are taken periodically over an interval time.

7. The procedure of claim 2 in which the more than one test breath samples are taken periodically over an interval of time.

8. The procedure of claim 5 in which the more than one test breath samples are taken periodically over an interval of time.

9. The procedure of claim 1 in which the test breath samples are taken about every 10 minutes for about 180 minutes.

10. The procedure of claim 2 in which the test breath samples are taken about every 10 minutes for about 180 minutes.

11. The procedure of claim 5 in which the test breath samples are taken about every 10 minutes for about 180 minutes.

12. The procedure of claim 1 including ingestion by the test subject of a meal to delay the emptying of the stomach so that the urease activity on the labeled urea is prolonged.

13. The procedure of claim 2 including ingestion by the test subject of a meal to delay the emptying of the stomach so that the urease activity on the labeled urea is prolonged.

14. The procedure of claim 5 including ingestion by the test subject of a meal to delay the emptying of the stomach so that the urease activity on the labeled urea is prolonged.

15. The procedure of claim 12 wherein the meal is liquid.

16. The procedure of claim 13 wherein the meal is liquid.

17. The procedure of claim 14 wherein the meal is liquid.

18. The procedure of claim 12 wherein the meal is solid.

19. The procedure of claim 13 wherein the meal is solid.

20. The procedure of claim 14 wherein the meal is solid.

21. The procedure of claim 12 wherein the meal contains the labeled urea.

22. The procedure of claim 13 wherein the meal contains the labeled urea.

23. The procedure of claim 14 wherein the meal contains the labeled urea.

24. The procedure of claim 1 wherein the carbon isotope is non-radioactive.

25. The procedure of claim 1 wherein the carbon isotope is radioactive.

26. The procedure of claim 2 wherein the carbon isotope is non-radioactive.

27. The procedure of claim 2 wherein the carbon isotope is radioactive.

28. The procedure of claim 5 wherein the carbon isotope is radioactive.

29. The procedure of claim 5 wherein the carbon isotope is non-radioactive.

30. A breath test procedure for detecting urease activity in a test subject's stomach and for determining the amount of such activity, the procedure comprising,

determining a baseline amount of naturally occurring $^{13}$carbon isotope in the test subject'a breath by taking and analyzing a pre-test breath sample from the test subject,

consumption by the subject of a meal, the meal including urea labeled with the carbon isotope, the urea present in an amount in the range of about 2.5 to about 10 milligrams per kilogram of weight of the test subject, the meal also including at least about 75 calories of nutrients to delay the emptying of the test subject's stomach and prolong the urease activity on the labeled urea,

collection of test breath sample from the subject about every 10 minutes for about 180 minutes after the meal has been consumed,

analysis of the test breath samples to determine the amount of the carbon isotope present in the exhaled carbon dioxide in the samples, and

determining the amount of the labeling carbon isotope.

31. A meal for use in a breath test for determining the presence of urease activity in the stomach of a test subject, the test subject having ingested an amount of carbon-isotope-labeled urea, the meal comprising,

liquid or solid nutrient material of at least about 75 calories,

said meal for delaying the emptying of the stomach and for prolonging the urease activity on the labeled urea.

TABLE I

TIME COURSE OF $^{13}CO_2$ RECOVERY IN 21 SUBJECTS AFTER ADMINISTRATION OF 5 mg/kg UREA-1-$^{13}C$

| Subject | 1 | 2 | 3 | 4 | 5 | 6 | 7 | 8 | 9 | 10 | 11 |
|---|---|---|---|---|---|---|---|---|---|---|---|
| Biopsy Result | | (-) | | (-) | (-) | | (-) | | | (-) | |
| MIN | CUMULATIVE PERCENT DOSE OF $^{13}C$ UREA RECOVERED AS $CO_2$ | | | | | | | | | | |
| 10 | -0.01 | 0.041 | 0.027 | 0.007 | -0.00 | -0.04 | 0.023 | 0.014 | 0.088 | 0.056 | na |
| 20 | -0.04 | 0.079 | 0.062 | 0.048 | -0.00 | -0.08 | 0.023 | 0.058 | 0.162 | 0.191 | na |
| 30 | -0.02 | 0.064 | 0.099 | 0.088 | 0.001 | -0.09 | 0.027 | 0.095 | 0.2 | 0.291 | 0.164 |
| 40 | -0.01 | 0.015 | 0.108 | 0.088 | 0.006 | -0.06 | 0.062 | na | 0.259 | 0.365 | 0.241 |
| 50 | -0.03 | -0.01 | 0.107 | 0.081 | -0.00 | -0.04 | 0.144 | 0.19 | 0.3 | 0.417 | 0.307 |
| 60 | -0.05 | -0.03 | 0.108 | 0.073 | -0.03 | -0.01 | 0.223 | 0.24 | 0.338 | 0.434 | 0.392 |
| 70 | -0.10 | -0.06 | 0.1 | 0.057 | -0.03 | -0.02 | 0.308 | 0.282 | 0.397 | 0.477 | 0.469 |
| 80 | -0.14 | -0.1 | 0.093 | 0.042 | -0.00 | -0.05 | 0.387 | na | 0.453 | 0.544 | 0.54 |
| 90 | -0.18 | -0.12 | 0.126 | 0.011 | 0.012 | -0.08 | 0.448 | na | 0.511 | 0.575 | 0.603 |
| 100 | -0.22 | -0.15 | 0.135 | -0.02 | 0.016 | -0.11 | 0.483 | 0.377 | 0.575 | 0.591 | 0.661 |
| 110 | -0.23 | -0.14 | 0.124 | -0.05 | -0.000 | -0.09 | 0.512 | 0.399 | 0.672 | 0.613 | 0.731 |
| 120 | -0.23 | -0.12 | 0.142 | -0.02 | 0.018 | -0.02 | 0.555 | 0.422 | 0.754 | 0.667 | 0.803 |
| 130 | -0.27 | -0.13 | 0.159 | 0.019 | 0.096 | 0.05 | 0.602 | na | 0.799 | 0.741 | 0.871 |
| 140 | -0.28 | -0.15 | 0.149 | na | 0.163 | 0.13 | 0.606 | na | 0.84 | 0.797 | 0.927 |
| 150 | -0.32 | na | 0.131 | 0 | 0.162 | 0.182 | 0.561 | 0.517 | 0.871 | 0.837 | 0.975 |
| 160 | -0.37 | -0.19 | 0.098 | 0.013 | 0.148 | 0.199 | 0.536 | 0.564 | na | 0.866 | 1.035 |
| 170 | -0.39 | -0.22 | 0.067 | 0.039 | 0.159 | 0.243 | 0.518 | 0.582 | 0.927 | 0.924 | 1.101 |
| 180 | -0.41 | -0.25 | 0.058 | 0.069 | 0.137 | 0.275 | 0.489 | 0.581 | 0.975 | 0.988 | 1.146 |

NA = Sample not analyzed

TABLE I (Cont'd...)

TIME COURSE OF $^{13}CO_2$ RECOVERY IN 21 SUBJECTS
AFTER ADMINISTRATION OF 5 mg/kg UREA-1-$^{13}$C

| Subject | 12 | 13 | 14 | 15 | 16 | 17 | 18 | 19 | 20 | 21 |
|---|---|---|---|---|---|---|---|---|---|---|
| Biopsy Result | (+) | (+) | | (+) | (+) | (+) | | (+) | | |

CUMULATIVE PERCENT DOSE OF $^{13}$C UREA
RECOVERED AS·CO$_2$

| MIN | | | | | | | | | | |
|---|---|---|---|---|---|---|---|---|---|---|
| 10 | 0.01 | 0.052 | 0.039 | 0.13 | 0.061 | 0.1 | 0.192 | 0.314 | 0.257 | 0.173 |
| 20 | 0.037 | 0.135 | 0.194 | 0.432 | 0.163 | 0.326 | 0.619 | 0.971 | 0.845 | 0.711 |
| 30 | 0.086 | 0.209 | 0.419 | 0.804 | 0.261 | 0.605 | 1.063 | 1.684 | 1.551 | 1.346 |
| 40 | 0.188 | na | 0.653 | 1.246 | 0.415 | 0.929 | 1.542 | 2.396 | 2.38 | 2.056 |
| 50 | 0.306 | na | 0.088 | 1.701 | 0.642 | 1.274 | 2.107 | 3.091 | 3.394 | 3.015 |
| 60 | 0.418 | 0.399 | 1.139 | 2.147 | 0.942 | 1.642 | 2.731 | 3.781 | 4.462 | 4.051 |
| 70 | 0.592 | 0.475 | 1.452 | 2.608 | 1.32 | 2.024 | 3.368 | 4.452 | 5.438 | 5.127 |
| 80 | 0.763 | 0.602 | 1.807 | 3.069 | 1.7 | 2.443 | 3.949 | 5.064 | 6.298 | 6.177 |
| 90 | 0.928 | 0.735 | 2.154 | 3.492 | 2.081 | 2.921 | 4.477 | 5.603 | 6.993 | 7.159 |
| 100 | 1.083 | 0.879 | 2.465 | 3.829 | 2.496 | 3.39 | 4.978 | 6.133 | 7.589 | 8.042 |
| 110 | 1.257 | 1.043 | 2.738 | 4.127 | 2.925 | 3.824 | 5.422 | 6.67 | 8.093 | 8.819 |
| 120 | 1.415 | 1.228 | 3.051 | 4.433 | 3.359 | 4.283 | 5.854 | 7.142 | 8.546 | 9.476 |
| 130 | 1.522 | 1.475 | 3.357 | 4.697 | 3.793 | 4.746 | 6.236 | 7.551 | 8.972 | 10.07 |
| 140 | 1.634 | 1.744 | 3.582 | 4.883 | 4.211 | 5.167 | 6.547 | 7.909 | 9.333 | 10.59 |
| 150 | 1.723 | 1.939 | 3.776 | 5.025 | 4.597 | 5.557 | 6.82 | 8.2 | 9.651 | 11.04 |
| 160 | 1.794 | 2.058 | 3.939 | 5.121 | 4.923 | 5.909 | 7.077 | 8.479 | 9.947 | 11.46 |
| 170 | 1.864 | 2.18 | 4.083 | 5.2 | 5.21 | 6.205 | 7.319 | 8.745 | 10.22 | 11.84 |
| 180 | 1.892 | 2.335 | 4.205 | 5.286 | 5.483 | 6.461 | 7.538 | 8.956 | 10.46 | 12.20 |

NA = Sample not analyzed

0 253 927

UREA BREATH TEST

C NOONAN   11 – 05 – 85

FIG. 1A

UREA BREATH TEST

C. N.        11 – 05 – 85

FIG. 1B

UREA BREATH TEST

ANTON O.    11-05-85

FIG.2

EFFECT OF BISMUTH THERAPY ON UREASE ACTIVITY

□   BEFORE
◇   1 DAY
△   10 DAY

FIG.7

## UREA BREATH TEST

### C. N.     11- 05 - 85

FIG.3A

## UREA BREATH TEST

### ANTON O.     11- 05- 85

FIG.3B

UREA BREATH TEST

BIOPSY RESULTS

FIG.4

UREA BREATH TEST

REPRODUCIBILITY

FIG.5

## UREA BREATH TEST # 01

### PDK 10-09-85

FIG.6A

## UREA BREATH TEST # 38

### PDK 02-01-86

FIG.6B

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,P | BIOLOGICAL ABSTRACTS, vol. 80, no. 5, 1986, page 1435, abstract no. 31056223, Philadelphia, US; D.Y. GRAHAM et al.: "Rapid noninvasive diagnosis of gastric campylobacter by a carbon-13 urea breath test", & GASTROENTEROLOGY (USA), 1986, 90(5, part 2, 1435 | 1-32 | G 01 N 33/497// C 12 Q 1/58 G 01 N 33/62 A 61 K 49/00 |
| | --- | | |
| X | US-A-3 772 436 (E. SHANBROM) * abstract, claims 1-3 * | 1,2 | |
| | --- | | |
| A | US-A-4 548 805 (D.A. SACK) * abstract, claim 1 * | 1,2,6, 7 | |
| | --- | | |
| A | US-A-4 298 347 (F.M. WALSH) * abstract * | 1,2 | |
| | --- | | |
| A | INTERNATIONAL JOURNAL OF APPLIED RADIATION AND ISOTOPES, vol. 34, no. 7, July 1983, pages 1013-1014, Oxford, GB; A.M. EMRAN et al.: "Preparation of C-urea from no-carrier-added C-cyanide" * page 1013, introduction * | 1,30 | |
| | ----- | | |

**TECHNICAL FIELDS SEARCHED (Int. Cl.4)**

G 01 N 33/00
C 12 Q 1/00
A 61 K 49/00
A 61 B 5/00

The present search report has been drawn up for all claims

| Place of search BERLIN | Date of completion of the search 30-04-1987 | Examiner DE KOK A.J. |
|---|---|---|